# EUROPEAN PATENT SPECIFICATION

(11) **EP 1 940 342 B1**
(45) Date of publication and mention of the grant of the patent: **23.08.2017**
(21) Application number: 06814189.4
(22) Date of filing: 05.09.2006
(51) Int. Cl.: A61K 6/083

(54) **DENTAL SEALANT COMPOSITIONS CONTAINING NANOMETER-SIZED SILICA**
DENTALVERSIEGELUNGSZUSAMMENSETZUNGEN MIT SILICA IN NANOMETER-GRÖSSE
COMPOSITIONS POUR SCELLEMENT DENTAIRE CONTENANT UNE SILICE NANOMETRIQUE

(30) Priority: 02.09.2005 US 713814 P
(43) Date of publication of application: 09.07.2008
(73) Proprietor: DENTSPLY International Inc., York, PA 17405-0872 (US)
(72) Inventor: WANG, Xiuling, Shirley, Bear, DE 19701 (US)
(74) Representative: Hartz, Nikolai
(86) International application number: PCT/US2006/034592
(87) International publication number: WO 2007/028159

(56) References cited:
- WO-A-01/30307
- WO-A-03/063804
- WO-A-2004/017928
- US-A- 5 936 006
- US-A1- 2002 065 337

## Description

### BACKGROUND OF THE INVENTION

### Field of the Invention

The present invention relates generally to dental compositions containing polymerizable resins and filler particles, particularly nanometer-sized silica particles. The dental compositions are dental sealants.

### Brief Description of the Related Art

Dental professionals use composite materials containing polymerizable resins and filler particles in many different applications because of their desirable properties. For example, composite materials can be used in crowns and bridges, fillings, and other restorations as well as sealants, adhesives, and cements. The composite material normally contains a polymerizable resin such as an acrylate or methacrylate resin; fillers such as silica and glasses; polymerization initiators such as 1, 2-diketone and dibenzoyl peroxide; polymerization accelerators such as tertiary amines; and various additives such as ultraviolet light absorbers, anti-oxidants, plasticizers, and the like. The composite can be self-cured or light-cured to form a hardened dental material that is strong and durable. Loading the composite with filler particles provides the material with good mechanical strength and durability. Some composite dental materials also have good aesthetic properties, particularly surface luster and translucency or opaque shading.

The dental industry is constantly looking to develop new compositions. In recent years, dental compositions containing polymerizable resins and "nanofiller" particles have been developed.

For example, Rheinberger et al., U.S. Patent 5,936,006 discloses a filled and polymerizable dental material, which can be used as a filling composite, fixing cement, or adhesive. The dental material is described as containing a sol of amorphous silica particles in a liquid, organic dispersion agent. The liquid, organic dispersion agent preferably contains at least one diol, at least one hydroxy (meth)acrylate, and at least one di(meth)acrylate, or mixtures thereof. It is particularly preferred that hexanediol diacrylate, 2-hydroxyethyl methacrylate, triethylene glycol dimethacrylate, bisphenol-A-glycidyl methacrylate, urethane dimethacrylate, or mixtures of these compounds be used. The silica particles are organically surface-modified with a treatment agent such as 3-(meth)acryloloxypropyl trialkoxy silane. The silica particles are further described as having an average particle size of 10 to 100 nm and being non-agglomerated.

Furman et al., U.S. Patent 6,194,481 discloses photocurable resins comprising zirconium oxide particles, which have been functionalized. The resins may be used for dental and medical restorations. The zirconium oxide particles have a nanometer-sized diameter which is small enough to provide sufficient transparency but large enough to provide the restoration with effective fracture toughness. The particles are treated with a silanating agent.

Wu et al., U.S. Patent 6,572,693 discloses dental materials, which can be used as adhesives, crowns, fillings, cavity liners, cements, orthodontic devices, prostheses, and sealants. The dental material comprises (a) a hardenable resin; and (b) a filler comprising: (i) clusters of nano-sized particles, and (ii) non-agglomerated, nano-sized particles. Suitable particles, which can be used as the nano-sized particles or clusters, are described as including non-heavy metal oxide particles such as silica, calcium phosphate, titanium oxide, feldspar, aluminum oxide, and the like. The clusters further include a heavy metal oxide, which imparts radiopacity to the material. Suitable heavy metal oxides are described as including the oxides of yttrium, strontium, barium, lanthanum, zirconium, tantalum, tin, and zinc.

Angeletakis et al., U.S. Patent 6,593,395 discloses a dental composite material, which can be used for dental restorations such as crowns, veneers, direct fillings, inlays, onlays, and splints. The composite includes a "ground structural filler" material having a mean particle size between about 0.05 µm and about 0.5 µm, and a "nanofiller" material, which comprises discrete, non-agglomerated particles with a mean particle size of less than 100 nm. Ground structural fillers are described as including aluminosilicate glass, aluminoborosilicate glass, silica, silica-zirconia, silica-titania, barium oxide, quartz, and alumina. Nanofillers are described as including powders such as aluminosilicate powder having a mean particle size of less than 80 nm.

Zhang et al., U.S. Patent 6,899,948 discloses dental materials comprising a hardenable resin and "nano-sized" silica particles dispersed within the resin. By "nano-sized," it is meant that the silica particles have an average diameter size of less than 200 nm. The nano-sized silica particles are further described as being discrete and non-agglomerated and in a dry powder form. The dental materials can be used as adhesives, crowns, fillings, cavity liners, cements, orthodontic devices, prostheses, and sealants according to the '948 Patent.

Document WO2004/017926 discloses a dental sealant (examples 13 and 14) comprising CQ in an amount between 3-7wt% and a mixture of acrylates. The composition can be applied to the surface of the tooth and the sealant if fully cured thanks to the no formation of an oxygen inhibition layer.

Although some of the foregoing dental compositions containing nanofiller or nanosized particles have some beneficial properties, there is still a need for an improved composition. Particularly, there is a need for an improved dental composition that can be used as a dental sealant.

Dental sealants provide long-term protection against dental caries, which is caused by the accumulation of bacteria. The bacteria in plaque produce acids that eat into the tooth, eventually causing cavities to form therein. Pits and fissures may develop on and in the surfaces of teeth, and bacteria tend to accumulate in these areas. Sealants are commonly used to fill the pits and fissures on the surfaces of teeth, particularly the occlusal surfaces of posterior teeth. The sealants provide a smooth seal and prevent the ingress of fluids, food, and debris. The sealant can either have a clear composition or opaque composition to match the natural color of the tooth.

Clear sealants also can be applied to the surfaces of composite restorations (for example, fillings) in order to improve the luster and aesthetic appearance of the restoration. If the dental professional applies the sealant to the composite restoration and obtains a surface having sufficient shine and gloss, he or she may not need to polish the restoration. Polishing the restoration can be a relatively lengthy procedure, requiring different instruments and polishing pastes. Eliminating the polishing step can make the dental treatment process more efficient for the patient and dentist. The amount of time, which the patient must spend in the dental chair is reduced upon eliminating the polishing step.

However, one problem with applying some conventional surface sealants over composite restorations is that the sealant may have low mechanical strength and poor wear-resistance. Over time, as the sealant wears away, the restoration loses its luster. This can lead to the tooth having a dull surface and non-aesthetic appearance. In such instances, the restoration must be polished periodically. Accordingly, there is a need for a sealant having good mechanical strength and wear-resistance that can be applied over composite restorations. The sealant should have good scratch/abrasion resistance so that it will not wear away easily. Moreover, the sealant should have high surface gloss so that the restoration appears shiny and new. The present invention provides a dental composition, which can be used as a sealant, and the sealant has these desirable properties as well as other features and advantages.

Another problem with some traditional sealant compositions is that they are difficult to fully cure and polymerize. Some compositions may not completely polymerize, since oxygen is present in the air. The reactive radicals, which are generated from the photoinitiators or monomers in the composition, react with the oxygen to form peroxy radicals. Such peroxy radicals are generally inefficient for initiating further polymerization of the composition. These undesired reactions with oxygen occur mainly at the interface between the air and composition (photo reaction media). The concentration of oxygen is higher at the air interface than the concentration of oxygen within the composition (photo reaction media). In such instances, the composition is considered to form an "air-inhibited layer" (AIL) on the surface of the restoration or tooth. As a result, the surface of the restoration or tooth feels sticky or tacky, and the surface gloss is low. Thus, there is a need for a sealant composition, which can be cured easily and completely. The sealant should be able to form a hard, non-sticky, glossy surface layer on the restoration or tooth. The present invention provides a dental sealant having these advantageous properties.

### SUMMARY OF THE INVENTION

The present invention provides a dental sealant as defined by the claims.

The present invention relates to an improved dental composition containing a polymerizable resin and nanometer-sized silica particles. The dental composition is used as a sealant. More particularly, the invention relates to a dental sealant composition for application to a surface of a tooth or dental restoration such as, for example, a composite filling material.

The method involves the steps of: a) providing a sealant composition comprising a filler system of colloidal silica particles dispersed within a methacrylate resin, wherein the particles have an average particle size of about 10 nm to about 100 nm, and a polymerization system capable of being activated by light; b) applying the sealant to a tooth surface or dental restoration surface; and c) irradiating the composition with light so that the composition hardens.

The polymerization system contains as photoactive agents camphorquinone (CQ) and 2,4,6 trimethylbenzoyldiphenyl phosphine oxide (TPO). Co-polymerization initiator, namely ethyl (4-N, N-dimethylamino) benzoate) is added to the composition. Preferably, the composition is cured by blue visible light having a wavelength in the range of about 400 nm to about 500 nm. The hardened sealant has good mechanical strength and durability. Sealants having substantially transparent or opaque shades can be made in accordance with this invention.

### DETAILED DESCRIPTION OF THE PREFERRED EMBODIMENTS

The present invention relates to a dental sealant composition for application to a surface of a tooth or dental restoration such as, for example, a composite filling material.

First, a sealant composition comprising a filler system and polymerization system is provided.

A polymerizable resin is provided in the filler system used to prepare the composition of this invention as discussed in further detail below. Additionally, the sealant composition may contain a polymerizable dental resin. Such resins are well known in the art and include, for example, polymerizable monomers such as ethylene glycol di(meth)acrylate, diethylene glycol di(meth)acrylate, triethylene glycol di(meth)acrylate, tetraethylene glycol di(meth)acrylate, neopentyl glycol di(meth)acrylate, 1,4-butanediol di(meth)acrylate, 1,6-hexanediol di(meth)acrylate, trimethylolpropane tri(meth)acrylate, 2,2-bis[4-(2-hydroxy-3-acryloyloxypropoxy)phenyl]propane, 2,2-bis[4-(2-hydroxy-3-methacryloyloxypropoxy)phenyl]propane (Bis-GMA), 2,2-bis[4-(acryloyloxy-ethoxy)phenyl]propane, 2,2-bis[4-(methacryloyloxy-ethoxy)phenyl]propane, urethane di(meth)acrylate (UDMA), alkoxylated pentacrythritol tetraacrylate, ethoxylated bisphenol A dimethacrylate, dipentaerythritol pentacrylate phosphoric acid ester (PENTA), bis[2-(methacryloxyloxy)-ethyl]phosphate, and the like.

In addition to the foregoing monomers and oligomers, the composition may contain diluent monomers such as hydroxy alkyl methacrylates, ethylene glycol methacrylates, and diol methacrylates such as tri(ethylene glycol) dimethacrylate (TEGDMA) to reduce viscosity and achieve suitable application properties. The monomers and oligomers are typically present in the composition in an amount in the range of about 10 % to about 90 % based on the total weight of the composition and preferably in an amount in the range of about 20% to about 60%.

The sealant composition of this invention contains a filler system comprising colloidial silica particles dispersed within methacrylate resins. Such resins include methacrylate monomers and oligomers monomers including, for example, tri(ethylene glycol) dimethacrylate (TEGDMA); hydroxyethyl methacrylate (HEMA); urethane di(meth)acrylate (UDMA); triethylenglycol dimethacrylate (TRGDMA); hyroxypropyl methacrylate (HPMA); hydroxyethyl methacrylate (HEMA); Bisphenol-A diglycidyl dimethacrylate/triethylenglycol dimethacrylate (Bis-GMA/TRGDMA); urethane dimethacrylate/triethylenglycol dimethacrylate (UDMA/TRGDMA); and 1, 4-butanediol dimethacrylate (1,4-BDDMA). The silica particles in the dispersion are preferably spherical-shaped and are surface-modified. The silica particles may be surface-modified by treating the particles with silane compounds. The individual particles have a diameter size in the range of about 10 nm to about 100 nm. More preferably, the silica particles have an average diameter size of about 20 nm and a narrow particle size distribution. The concentration of silica particles in the dispersion may vary, but it is typically in the range of about 10 wt.% to about 60 wt.%. Such filler materials are available from Hanse Chemie under the tradename, NANOCRYL. The silica particles are non-agglomerated and distributed homogeneously within the resin matrix. As a result, the dispersion has a relatively low viscosity and is free-flowing even when the content of silica particles in the dispersion is as high as 60 wt.%.

The composition further contains a polymerization system. Preferably, the composition is cured by blue visible light having a wavelength in the range of about 400 to about 500 nm. The polymerization system contains as photoactive agent camphorquinone (CQ), which has an absorbence maximum between 400 and 500 nm. Co-polymerization initiator, namely ethyl (4-N, N-dimethylamino)benzoate) is added to the composition. Other photoactive agents that are used include 2,4,6 trimethylbenzoyldiphenyl phosphine oxide (LucirinTPO).
The phosphine oxide initiators are capable of generating free radicals for polymerization when irradiated with light at wavelengths in the range of about 400 to about 1200 nm. The polymerization initiators are typically present in the composition in the range of about 0.5 to about 10 wt.% and preferably in the range of about 1 to about 10 wt.%.

The dental composition of this invention may further contain conventional inorganic fillers in addition to the above-described nanometer-sized silica particles. Examples of conventional fillers include glass, quartz, barium borosilicate, strontium borosilicate, borosilicate, barium silicate, strontium silicate, lithium silicate, lithium alumina silicate, calcium phosphate, alumina, zirconia, tin oxide, titanium dioxide, and the like. Such conventional fillers typically have a particle size in the range from about 0.1 to about 5.0 microns and are silane-treated. It may be desirable to add such larger-sized filler particles to the dispersion, because they create interstitial spaces or voids in the resin matrix, which can be filled by the nanometer-sized particles. In this manner, the total filler content can be increased and this may further enhance the strength and other mechanical properties of the composition. The total amount of filler particles in the composition is typically in the range of about 10% to about 70% based on the total weight of the composition and preferably in the range of about 20% to about 60%.

The compositions of this invention can be used as various dental materials including, for example, crown and bridge materials, adhesives, cements, fillings, sealants, inlays, onlays, veneers, and the like. Preferably, the composition is used as a dental sealant for sealing pits and fissures on and in the surface of a restoration or tooth. The dental sealant may be applied to the surface of a tooth using methods well known in the art. First, the surface of the tooth is cleaned thoroughly. Prophylaxis pastes can be used to clean the surface of the tooth. Secondly, the surface of the tooth is dried. The surface of the tooth can be dried with cotton rolls, an air syringe, or other appropriate materials. Thirdly, the surface of the tooth is acid-etched. A liquid etchant can be brushed onto the surface of the tooth to prepare the tooth. The tooth is dried after it has been acid-etched. Fourthly, the sealant is applied to the etched surface of the tooth with a brush or other suitable applicator. Finally, the sealant may be cured to the tooth by irradiating it with visible blue light. A standard dental light-curing unit may be used to cure the sealant composition. The resulting dental sealant shows good mechanical strength and wear-resistance. Furthermore, the sealant provides a hard, smooth and glossy surface finish over the tooth.

The invention is further illustrated by the following Examples using the below-described Test Methods, but these Examples should not be construed as limiting the scope of the invention.

### Test Methods

### Vickers Hardness

The sealant materials were tested for Vickers Hardness according to the following procedures. The sealant material, which was to be tested, was first placed in a cylindrical mold measuring 20 mm (diameter) x 2 mm (height) and cured with a TRIAD 2000 visible light curing unit (Dentsply) for two minutes. The cured disk-shaped specimens then were tested for hardness using LM700AT Digital Microhardness equipment (LECO Corp.) under 10 gms load and 10 seconds dwell time.

### Contrast Ratio

The contrast ratios of the sealant materials were tested according to the following procedures. The sealant material, which was to be tested, was first placed in a cylindrical mold measuring 30 mm (diameter) x 1 mm (height) and cured with a TRIAD 2000 visible light curing unit (Dentsply) for two minutes. The cured disk-shaped specimens were evaluated to determine the amount of light that was transmitted through the disk by measuring contrast ratio. A Macbeth^{®} Color-Eye^{®} (Gretag Macbeth) was used to measure the contrast ratio of the samples.

### Flexural Strength

The sealant materials were tested for Flexural Strength according to the following procedures. The sealant material, which was to be tested, was first placed in a 2 mm x 2 mm x 25 mm mold and cured with a SPECTRUM 800 visible light-curing unit (Dentsply) at 550 mW/cm² for twenty seconds. The cured specimens were tested for flexural strength using standard ISO 4049 method.

### Prophy Wear-Resistance

The sealant materials were tested for prophy wear-resistance according to the following procedures. The sealant material, which was to be tested, was first placed in a cylindrical mold measuring 20 mm (diameter) x 2 mm (height) and cured with a TRIAD 2000 visible light curing unit (Dentsply) for two minutes. The disc-shaped specimens were placed under a restraining clamp in a prophy wear-resistance machine (Dentsply), and the mounted specimens were polished with coarse prophy paste. A prophy cup, which was attached to a rotatable polishing head, was filled with coarse prophy paste. The machine was counter-balanced using a 500-gram weight. The polishing head rotated and polished the specimens with the coarse prophy paste. The total time period of the polishing cycle was 5 minutes and 30 seconds. Upon completion of the polishing cycle, the specimens were washed with water. The wear for each sample was reported as a volume loss (mm³), which was determined by a prophilometer (Taylor-Hobson).

### Adhesion Strength

The sealant materials were tested for adhesive strength according to the following procedures. A composite dental restorative material (Esthet-X from Dentsply), which was to be coated with the sealant, was first placed in a cylindrical mold measuring 20 mm (diameter) x 10 mm (height) and cured with a TRIAD 2000 visible light curing unit (Dentsply) for two minutes. Then, the surfaces of the disc-shaped composite substrates were sanded with 600 grit abrasive paper and gently dried. The sealant composition was then applied to the surfaces of the cured, composite substrate. Next, the sealant-coated surface was blown dry and light-cured cured with a Spectrum 800 visible light-curing unit (Dentsply) at 550 mW/cm² intensity for ten seconds. Gelatin capsule posts (4.5 mm in diameter) were filled with a composite resin TPH spectrum (Dentsply) and then positioned onto the sealant-coated surface area. The excess flashing, from the capsule posts, was gently removed using a dental explorer and the resin-containing posts were light-cured a with Spectrum 800 visible light-curing unit at 550 mW/cm² intensity for 20 seconds to adhere the posts to the sealant-coated surface. The substrate samples with bonded gelatin posts were placed in a 37 °C distilled water bath for twenty-four hours before testing. The samples were removed from the bath and the shear bond strength of the sealant to the substrate was tested (in compressive shear mode) using an Instron.

### Examples

The following table provides a list of the chemical compounds used to prepare the compositions in the Examples:

| Product | Chemical Name | Commercial Source |
|---|---|---|
| PENTA | Dipentaerythritol pentacrylate phosphoric acid ester | Dentsply |
| Bis-HEMA phosphate | Bis[2-(methacryloxyloxy)-ethyl]phosphate | Aldrich |
| Nanocryl D120 * | 50 wt %. of SiO2, 20 nm particle size, dispersed in alkoxylated (4) pentaerythritol tetraacrylate | Hanse Chemie |
| Nanocryl D322 | 50 wt %. of SiO2, 20 nm particle size, dispersed in urethane dimethacrylate (80 wt%) and triethylene glycol dimethacrylate | Hanse Chemie |
| Nanocryl XP21/0568 | 50 wt %. of SiO2, 20 nm particle size, dispersed in triethylene glycol dimethacrylate | Hanse Chemie |
| Nanocryl XP/0746 | 50 wt %. of SiO2, 20 nm particle size, dispersed in hydroxyethyl methacrylate | Hanse Chemie |
| Nanocryl XP 21/1045 * | 50 wt %. of SiO2, 20 nm particle size, dispersed in trimethylopropane triacrylate | Hanse Chemie |
| Nanocryl D301 | 50 wt %. of SiO2, 20 nm particle size, dispersed in triethylene glycol dimethacrylate | Hanse Chemie |
| CQ | Camphorquinone | Hamford Research |
| EDAB | Ethyl-(4-N,N-dimethylamino) benzoate | Aldrich |
| Lucirin TPO | 2,4,6-trimethylbenzoyldiphenylphosphine oxide | Aldrich |

| | | |
|---|---|---|
| * (REFERENCE) | | |

### Example 1 (REFERENCE)

A transparent liquid mixture, which would be used as a dental surface sealant, was prepared by blending 9.5 gms of Nanocryl D120 and 0.5 gms of Lucirin TPO at 50 °C. The liquid mixture was tested for physical and chemical properties according to the above test methods, and the results are reported below in Tables 1-4. The mixture also was painted on an Esthet-X composite dental restorative (Dentsply) and light-cured with a Spectrum 800 visible light-curing unit (Dentsply) at 500 mW/cm² intensity for twenty seconds. Afterwards, a laboratory technician touched the sealant-coated surface of the composite restorative with a finger to test the surface for tackiness. The sealant-coated surface was found to be non-tacky and hard and had high surface gloss. No air-inhibition layer (AIL) was detected on the sealant-coated surface.

### Example 2

A transparent mixture, which would be used as dental surface sealant, was prepared by blending 9.74 gms of Nanocryl D322, 0.01gms of CQ, 0.05 gms ofEDAB, and 0.2 gms of Lucirin TPO at 50 °C. The liquid mixture was tested for physical and chemical properties according to the above test methods, and the results are reported below in Tables 1-4. The liquid mixture also was painted on an Esthet-X composite dental restorative (Dentsply) and light-cured with a Spectrum 800 visible light curing unit at 500 mW/cm² intensity for twenty seconds. The sealant-coated surface of the composite restorative was found to be slightly tacky.

### Example 3

A transparent mixture, which would be used as dental surface sealant, was prepared by blending 2.5 gm of PENTA, 1.8 gm of Nanocryl XP21/0568, 1.5 gm of Up21/0746, 2.5 gm of Nanocryl D322, 0.5 gm of Nanocryl XP1045, 1.0 gm of bis-hema phosphate, 0.02gm of CQ, 0.08gm of EDAB, and 0.1 gm of Lucirin TPO. The liquid mixture was tested for physical and chemical properties according to the above test methods, and the results are reported below in Tables 1-4. The liquid mixture was painted on an Esthet-X composite dental restorative (Dentsply) and light-cured with a Spectrum 800 visible light curing unit at 500 mW/cm² intensity for twenty seconds. The sealant-coated surface of the composite restorative was found to be slightly tacky.

### Comparative Example A

A commercially-available dental sealant, BISCOVER, from Bisco, Inc. was tested for various physical and chemical properties in accordance with the above test methods and the results are reported below in Tables 1-4.

### Comparative Example B

A commercially-available dental sealant, SEAL-N-SHINE, from Pulpdent Corp. was tested for various physical and chemical properties in accordance with the above test methods and the results are reported below in Tables 1-4.

### Comparative Example C

A commercially-available dental sealant, OPTIGARD, from Kerr Manufacturing (Sybron Dental Specialties, Inc.) was tested for various physical and chemical properties in accordance with the above test methods and the results are reported below in Tables 1-4.

**Table 1**

| **(Contrast Ratio)** | | | | | | |
|---|---|---|---|---|---|---|
| | Biscover | Seal-n-Shine | Optigard | Example 1 * | Example 2 | Example 3 |
| Contrast Ratio (%) | 13.42 | 12.42 | 12.88 | 12.52 | 13.89 | 12.9 |

| | | | | | | |
|---|---|---|---|---|---|---|
| * REFERENCE | | | | | | |

**Table 2**

| **(Vickers Hardness)** | | | | | | |
|---|---|---|---|---|---|---|
| | Biscover | Seal-n-Shine | Optigard | Example 1 * | Example 2 | Example 3 |
| Vickers Hardness | 13.6 ± 0.5 | 9.4 ± 1.1 | 11.2 ± 0.4 | 27.8 ± 1.3 | 31.0 ± 1.0 | 30.8 ± 3.3 |

| | | | | | | |
|---|---|---|---|---|---|---|
| * REFERENCE | | | | | | |

**Table 3**

| **(Wear-Resistance)** | |
|---|---|
| Wear Resistance (Prophy Abrasion) | Volume Loss (mm³) |
| Biscover | 8.88 ± 1.92 |
| Seal-n-Shine | 9.60 ± 0.85 |
| Optigard | 12.68 ± 1.24 |
| Example 1 * | 2.21 ± 0.14 |
| Example 2 | 2.73 ± 0.35 |
| Example 3 | 2.80 ± 1.1 |

| | |
|---|---|
| * REFERENCE | |

**Table 4**

| **(Adhesion)** | | | | | | |
|---|---|---|---|---|---|---|
| Substrate | 24 Hrs SBS (Mpa) | | | | | |
| | Biscover | Seal-n-Shine | Optigard | Example 1 * | Example 2 | Example 3 |
| Cured Composite (Esthet-X) | 20.6 ± 3.9 | 26.6 ± 4.5 | 21.4 ± 4.7 | 24.1 ± 4.2 | 25.2 ± 1.3 | 29.22 ± 1.9 |

| | | | | | | |
|---|---|---|---|---|---|---|
| * REFERENCE | | | | | | |

Referring to Table 1, the Contrast Ratios of the sealant compositions of this invention and commercially-known sealants are provided. The number values reported in Table 1 are visual opacity percentages, representing the visual opacity of the samples as light is transmitted through the sample during the testing method. The greater the visual opacity percentage means the greater the opacity of the composition. In compositions having low visual opacity percentages, more light is able to pass through the composition. In compositions having high visual opacity percentages, less light is able to pass through the composition. As shown in Table 1, the sealant compositions of this invention have a visual opacity of less than 14%. This means that the sealants of this invention have good light transparency and can provide a clear, clean surface layer over the restoration or tooth.

Table 2 provides information about the hardness of the sealants of this invention versus commercially-known sealants. The reported hardness values indicate the composition's resistance to being deformed when a load is applied to the composition. The greater the reported numerical value in Table 2 means the greater the hardness of the sealant and resistance of the sealant to deformation. As shown in Table 2, the sealant compositions of this invention have hardness values in the range of about 26 to about 35 in contrast to commercially-known sealants which have hardness values in the range of about 8 to about 14. The sealants of this invention have high hardness and mechanical strength.

In Table 3, the wear-resistance properties of the sealants of this invention and commercially-known sealants are reported. The wear-resistance of each sample is reported as a change in volume of the sample after it has been tested in accordance with the above testing methods. In samples having relatively high volume-loss numerical values, the wear-resistance of the composition is poor; the composition tends to wear away during testing. In samples having relatively low volume-loss numerical values, the wear-resistance of the composition is good; the composition does not wear-away during testing. As shown in Table 3, the sealants of this invention have high wear-resistance versus conventional sealants.

Lastly, referring to Table 4, the adhesive strength properties of the sealant compositions are reported. The reported adhesive strength values indicate the composition's ability to bond to a composite dental restoration (Esthet-X from Dentsply). The greater the reported numerical value in Table 4 means the greater the adhesive strength of the sealant composition.

## Claims

1. A dental sealant for application to a surface of a tooth or dental restoration, said composition comprising
(i) a filler system having colloidal silica particles dispersed within a methacrylate resin, said particles having an average particle size in the range of 10 nm to 100 nm; and
(ii) a polymerization system capable of being activated by light, which comprises photoactive agents in an amount of from 1 to 10 wt.-% based on the total amount of the composition, wherein the photoactive agents are camphorquinone, 2,4,6 trimethylbenzoyldiphenyl phosphine oxide and ethyl (4-N, N-dimethylamino) benzoate.

2. The dental sealant of claim 1, wherein the composition is cured by blue visible light having a wavelength in the range of about 400 nm to about 500 nm.

3. The dental sealant of claim 1, wherein the sealant composition is substantially transparent.

4. A method of applying a dental sealant to a surface of a dental restoration or tooth, comprising the steps of:
a) providing a dental sealant according to any one of claims 1 to 3;
b) applying the sealant composition to a surface of a tooth or dental restoration; and
c) irradiating the sealant composition with light so that the composition hardens,
whereby methods for treatment of the human or animal body by surgery or therapy are excluded.

## Patentansprüche

1. Dentalversiegelung zum Aufbringen auf eine Oberfläche eines Zahns oder einer Zahnrestauration, wobei die Zusammensetzung folgendes umfasst,
(i) ein Füllstoffsystem, das kolloidale Silicapartikel aufweist, die in einem Methacrylatharz dispergiert sind, wobei die Partikel eine durchschnittliche Partikelgröße in einem Bereich von 10 nm bis 100 nm aufweisen; und
(ii) ein Polymerisationssystem, das durch Licht aktiviert werden kann, welches photoaktive Mittel in einer Menge von 1 bis 10 Gewichtsprozent, basierend auf der Gesamtmenge der Zusammensetzung umfasst, wobei die photoaktiven Mittel Campherchinon, 2,4,6-Trimethylbenzoyldiphenylphosphinoxid und Ethyl(4-N,N-Dimethylamino)benzoat sind.

2. Dentalversiegelung nach Anspruch 1, wobei die Zusammensetzung durch blaues sichtbares Licht mit einer Wellenlänge in einem Bereich von 400 nm bis etwa 500 nm gehärtet wird.

3. Dentalversiegelung nach Anspruch 1, wobei die Versiegelungszusammensetzung im Wesentlichen transparent ist.

4. Verfahren zum Aufbringen einer Dentalversiegelung auf eine Oberfläche einer Zahnrestauration oder eines Zahns, umfassend die folgenden Schritte:
a) Bereitstellen einer Dentalversiegelung nach einem der Ansprüche 1 bis 3;
b) Aufbringen der Versiegelungszusammensetzung auf die Oberfläche eines Zahns oder einer Zahnrestauration; und
c) Bestrahlen der Versiegelungszusammensetzung mit Licht, so dass die Zusammensetzung härtet, wobei Verfahren zur Behandlung des menschlichen oder tierischen Körpers durch chirurgische oder therapeutische Behandlung ausgeschlossen sind.

## Revendications

1. Agent de scellement dentaire destiné à être appliqué à une surface d'une dent ou d'une restauration dentaire, ladite composition comprenant
(i) un système de charge ayant des particules de silice colloïdale dispersées dans une résine de méthacrylate, lesdites particules ayant une taille de particule moyenne se trouvant dans la plage allant de 10 nm à 100 nm ; et
(ii) un système de polymérisation capable d'être activé par la lumière, qui comprend des agents photoactifs en une quantité allant de 1 à 10% en poids par rapport à la quantité totale de la composition, où les agents photoactifs sont la camphorquinone, l'oxyde de 2,4,6-triméthylbenzoyldiphénylphosphine et le (4-N,N-diméthyl-amino)benzoate d'éthyle.

2. Agent de scellement dentaire de la revendication 1, dans lequel la composition est durcie par une lumière visible bleue ayant une longueur d'onde se trouvant dans la plage allant d'environ 400 nm à environ 500 nm.

3. Agent de scellement dentaire de la revendication 1, dans lequel la composition de l'agent de scellement est essentiellement transparente.

4. Procédé d'application d'un agent de scellement dentaire à une surface d'une restauration dentaire ou d'une dent, comprenant les étapes qui consistent :
a) à fournir un agent de scellement dentaire selon l'une quelconque des revendications 1 à 3 ;
b) à appliquer la composition de l'agent de scellement à une surface d'une dent ou d'une restauration dentaire ; et
c) à irradier la composition de l'agent de scellement avec la lumière de sorte que la composition durcisse,
moyennant quoi des procédés pour le traitement du corps humain ou animal par chirurgie ou thérapie sont exclus.
